# EUROPEAN PATENT APPLICATION

(11) **EP 2 749 636 A1**
(43) Date of publication of application: **02.07.2014**
(21) Application number: 12199477.6
(22) Date of filing: 27.12.2012
(51) Int. Cl.: C12M 1/107, C04B 28/00, C04B 28/08

(54) **Method of using alkali-activated non-cement binders for producing biogas from cheese whey**

(71) Applicant: Rigas Tehniska universitate, 1658 Riga (LV)
(72) Inventor: Rugele, Kristine, 1016 Riga (LV); Skripsts, Eriks, 3113 Plienciems (LV); Bajare, Diana, 1004 Riga (LV); Larsson, Simona, 1010 Riga (LV); Juhna, Talis, 1006 Riga (LV)
(74) Representative: Fortuna, Jevgenijs

(57) **Abstract**

The invention relates to method for producing biogas from cheese whey using alkali-activated-non-cement binders. The use of a cement free alkali activated binder comprising alumo-silicate source materials, lead-silicate glass, calcinated metakaolin clay, and alcaline inorganic materials comprising sodium materials as pH stabilizer and/or material for cell immobilization for producing biogas from cheese whey is suggested. The invention allows to considerably increase the rate of biogas production from fermentation medium containing cheese whey as well as to utilize waste from aluminium scrap recycling industry, calcined metakaolin and glass waste.

## Description

### Technical Field

The invention relates to methods for producing biogas from cheese whey, more particularly, to methods for producing biogas from cheese whey using alkali-activated-non-cement binders.

### Background Art

Production of biogas from waste has become topical nowadays (Directive 2009/28/EC of the European Parliament and of the Council). There are many inventions in the field of utilizing different type of waste for producing biogas known. In some cheese processing enterprises and dairy farms cheese whey is considered a waste water and it is not being processed in an effective way.

More than 40% of the European Union (EU) milk is processed into cheese. There are many varieties of cheese resulting in different cheese making technologies, but in average the final volume of whey is about 85-90 % of the volume of the processed milk. Whey is used directly as animal feed or used as field fertilizer, but usage of whey for energy production is not widespread. Cheese whey has sufficient biogas potential however it is a complicated substrate for biomethane production due to the process instability. The main components of cheese whey are: lactose (44-52 g. 1-1), protein (6.1-6.6 g. 1-1), fat (0.2-0.3 g. 1-1) and minerals (5-7.9 g· 1-1) [7]. Sweet whey having a pH of >5.6, results from rennet-coagulated cheese manufacture. Although the composition of each whey type is somewhat different and variable, both sweet and acid whey contain about 0.7% to 0.8% protein on a liquid basis, with whey proteins only representing about 10% to 12% of the total solids of whey [14]. Dairy waste water contains complex organics, such as polysaccharides, proteins and lipids, which after hydrolysis form sugars, amino acids, and fatty acids [5; 27]. In subsequent acidogenic reaction, these intermediate products are converted to volatile fatty acids (VFA), which are further degraded by acetogens, forming acetate, CO₂, and H₂. Lastly, both acetate and H₂/CO₂, are converted by methanogens to CH₄. The acid accumulation is due to the slower growth rate of the microbes that utilize the acetic and propionic acids than the rest of the microbial population in the reactor; therefore if the microbes produce acids faster than is the capacity of acid utilization, there will be an accumulation of acids, and consequently, a drop in pH, unless the system is well buffered. Another problem is hydrogen accumulation, which can lead to the inactivation of the reaction that produces acetate from propionate [22].

The addition of alkali is the most common remedy when a reactor is going "sour", namely there is a drop in pH due to formation of various acids. Another possible solution is using a sorbent (binder) with appropriate qualities.

Some inventors have suggested using cheese whey for producing biogas. For instance, there has been a method described for producing biogas using liquid manure and cheese whey [12]. Manure is also a popular material for producing biogas [16]. All these materials however follow the same fermentation scheme which might require pH adjustment at a certain stage.

The lack of buffering capacity and the using of adding of alkali containing geopolymer is the main advantage of the proposed method. The main drawbacks of the known methods are the lack of buffering capacity and the using of alkali adding (pH regulation) either in the beginning or even during the fermentation to sustain the pH required for methanogenesis (minimum pH 7).

There are known alkali activated binders [6] used in different fields of industry - like non-cement concrete of an environmentally sustainable construction. Hardjito et al. [9] have demonstrated the useful engineering properties of non-cement concrete using fly ash and an alkaline liquid containing sodium silicate and sodiumhydroxide solution for structural applications. The alkaline liquids used for this type of concrete are still commercially manufactured, therefore additional industrial pollution is appearing. Deevasan and Ranganath [4] had idea that the non-cement technology could be more environmentally friendly if the alkaline solution could be replaced with industrial waste. Therefore they made experiments with alkaline solution from poly-fibre industries and conclude that it is possible to produce sorbent concrete of strength up to 55 MPa using industrial (waste) by-products such as low calcium fly ash and effluent alkaline solution. Natural minerals and waste materials have been investigated as sources of sorbents in recent years [1; 3; 18; 24; 25; 26; 28]

None of these authors describe use of aluminium scrap recycling waste as raw material for alkali activated, non-cement binders. However, the consumption of aluminium scrap recycling waste is rising continuously worldwide, which is great stimulus for developing a non-waste technology [21; 13; 19; 20].

The recovery of metal from black or white dross in aluminium recycling industry is done through the conventional metallurgical process all over the world. Residual aluminium recycling waste containing alumina, salts, impurities and a small amount (3-5%) of metallic aluminium was obtained during the second recovery process. The given type of waste may have different names in various scientific publications, for example: non-metallic product, the aluminium recycling, the residual oxide mix, the residues and even recycled or residual dross [2; 10; 23; 29; 8; 15]. It is generally considered that non-metallic product is a process waste and subject to disposal after metal has been recovered from dross.

### Disclosure of the Invention

The goal of the present invention is to provide a method for overcoming drawbacks of the prior art and producing biogas from cheese whey in a more efficient way. The set goal is achieved by using of a cement free alkali activated binder as pH stabilizer and/or material for cell immobilization for producing biogas from acid cheese whey. Said binder comprises alumo-silicate source materials, lead-silicate glass, calcinated metakaolin clay, and alcaline inorganic materials comprising sodium materials.

On the one hand the invention allows to utilize waste from aluminium scrap recycling industry and calcined metakaolin as well as glass waste as basic raw materials for production of the cement free alkali activated binder and subsequent producing biogas. On the other hand the invention allows to considerably increase the rate of biogas production from fermentation medium containing cheese whey.

### Brief Description of Drawings

Fig. 1 shows pictures of microstructure of hardened alkali activated, non-cement binder sample observed with SEM;
Fig. 2 shows diagram of obtaining biogas with and without the use of alkali-modified geopolymer.

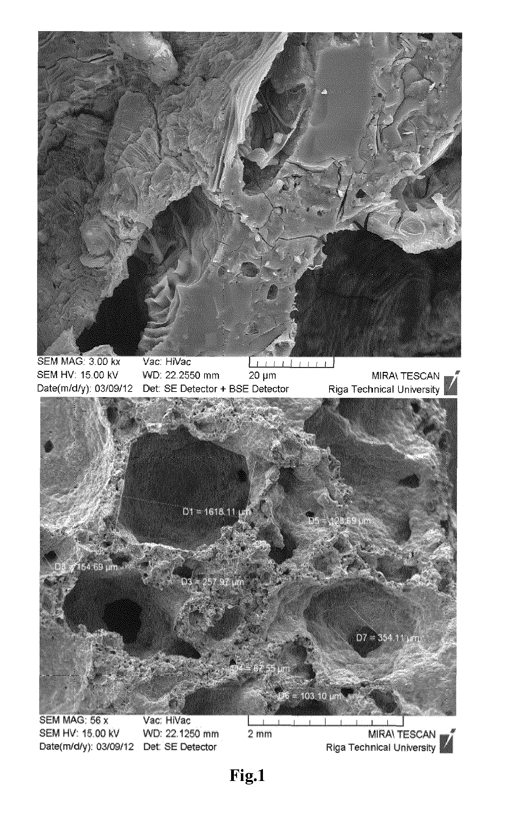

In the course of experiments a geopolymer - porous alkali activated, non-cement binder made from aluminium recycling waste has been created (Fig. 1). Aluminium recycling waste used was dross being impurities removed from the molten metal in scrap metal recycling process. Composition of porous alkali activated, non-cement binder material obtained consisted of aluminium recycling waste, recycled flourescent lamp lead-silicate glass, calcinated metakaolin clay, as well as commercially available alkali flakes (NaOH) and liquid glass (Na₂SiO₃+nH2O). Alkali activation of raw material composition was done by modifying water glass with an addition of sodium hydroxide to increase alkali solution concentration.

However in the light of the present invention it will be obvious to the skilled person that it is possible to use other embodiments of alkali activated non-cement binders as pH stabilizers and/or materials for cell immobilization for producing biogas from acid cheese whey.

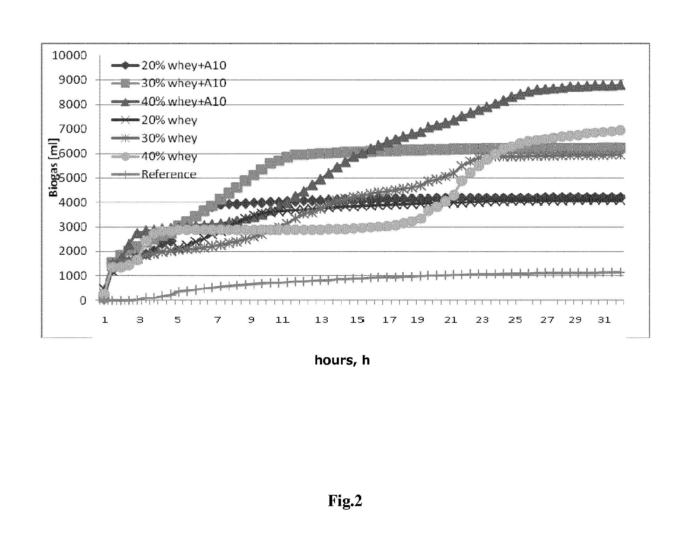

The geopolymer (porous alkali activated, non-cement binder made from aluminium recycling waste) according to the invention increases the rate of methane production compared to the experiments with no geopolymer added (Fig. 2). The nutrients were consumed by the day 10-13 and the maximum methane yield was achieved whereas the experiments with no geopolymer added still reached only 95% of biogas yield on day 23. The geopolymer addition showed the best results with increased organic loads.

The geopolymer used according to the invention was obtained as follows.

Preparation of solution for alkali activation: Sodium hydroxyl flakes were dissolved in the water glass (Na₂SiO₃) characterized by the silica modulus Ms = 3.3-3.5. Amount of sodium hydroxyl flakes was from 7 to 12% from the weight % of water glass, preferably from 9 to 11%.

### Preparation of raw materials.

*Component A.* The chemical composition of waste glass was as follows: SiO₂ - 67-70%, PbO - 19-23%, Al₂O₃ - 1-4%, Fe₂O₃ - 0.1-1%, CaO - 1-4%, Na₂O -7-10% and K₂O - 1-3%. In order to increase chemical activity and fineness of waste glass it was additionally grounded. The applied grinding period was 30 minutes to ensure homogeneity and fineness of waste glass particles. The ground waste glass contained particle sizes: from 8 µm up to 30 µm with the average grain size of 26.3 µm. The circularity coefficient of particles is 0.868, convexity coefficient - 0.978 and elongation coefficient - 0.335.

*Component B.* The chemical composition of aluminium recycling waste was as follows: metallic aluminium (Al) 3-15%, aluminium oxide (Al₂O₃) 20-50%, aluminium hydroxide (Al(OH)₃) 20-50%, aluminium nitride (AlN) 3-15%, silica (SiO₂) 3-30%, ferrous III oxide (Fe₂O₃) 2-20%, ferrous II oxide (FeO) 2-20%, magnum oxide (MgO) 1-20%, calcium oxide (CaO) 1-20%, others. The applied grinding period was 30 minutes to ensure homogeneity and fineness of aluminium recycling waste particles.

*Component C.* Kaolinite clay was calcinated in the temperature of 800°C. The calcination process of metakaolin was approximately 3 h long with temperature increase of 15°C/min and holding time at the maximum temperature (800°C) was 1 hour or by using a rotary furnace with calcination time no longer than 15 min and maximum temperature 700°C. Both of these calcination methods require grinding of the agglomerated products, therefore metakaolin was ground for 30 min.

Mixing. The composition of mixture: component A - 1 part by weight, component B-- 1 part by weight, component C - 1 part by weight. The components were mixed in the dry stage and solution for alkali activation was added in the amount 1.5- 2.5 parts by weight.

Curing conditions of the product were kept constant at the temperature regime of 80°C for 24h. The product was granulated in the granules with diameter 1 cm ± 3 mm.

Density of the obtained binder was from 400 to 900 kg/m³ and total porosity was from 60 to 85%. Compression strength of the binder was in the range from 1 to 4MPa. The pores serve for microorganism attachment, thus immobilization. These also protect the microorganisms from the influence of fermentation environment.

Further, influence of the binder material on cheese whey fermentation was investigated. It was surprisingly found to increase biogas yield, the effect being more pronounced at higher organic loads (OLR, organic loading rate). Alkali activated material aids to stabilize pH, especially at high OLRs, where the destabilizing effect of pH decrease is more pronounced.

Biogas fermentation proceeds in 4 stages, of which there is (i) hydrolysis; (ii) acetogenesis; (iii) acidogenesis and (iv) methanogenesis. Methanogenic organisms are very sensitive to pH decrease even if acids are used in methane formation. The same acids cause pH decrease if the system is not well buffered (such as acid cheese whey). The inventors found that said binder can serve both as pH stabilizer and as material for cell immobilization, thus serving two important factors towards successful biogas fermentation using acid cheese whey as a substrate.

The proposed method for producing biogas using said binder comprises the following steps:
(i) providing fermentation medium containing:
   - between 50 weight % and 80 weight %, preferably 55-65 weight % of inoculum,
   - between 20 weight % and 50 weight %, preferably 35-45 weight % of cheese whey;
(ii) adding 10-20 weight %, preferably 15 weight % of a cement free alkali activated binder (the amount of said binder is being calculated as 10-20 weight percent of the total weight of said inoculum and cheese whey),
(iii) performing fermentation,
(iv) collecting the produced biogas.

The inoculum can be organic origin substrates, such as vegetable raw materials (e.g. rape, cornstalk), manure (e.g. cow manure), food waste and other.

The cheese whay can be acid cheese whay having FOS/TAC (the contents of Volatile Organic Acids (FOS) and the buffer capacity (TAC)) between 3000 mg/L and 13000 mg/L.

The non-restrictive example of possible characteristics of the inoculum and acid cheese whey is shown in Table 1.

**Table 1. The example of characteristics of the inoculum and acid cheese whey.**

| **Parameter** | **Inoculum** | **Whey** |
|---|---|---|
| pH (20 °C) | 8.01 | 4.72 |
| Total solids (% w/w) | 2.86 | 5.68 |
| Volatile solids (% w/w) | 1.62 | 4.92 |
| COD_{unfiltred} (mg O₂/L) | - | 73.3 |
| COD_{filtred} (mg O₂/L | - | 29.5 |
| Ash (% w/w) | 0.59 | 0.69 |
| Protein (% w/w) | - | 0.3 |
| NPN (% w/w) | - | 0.2 |
| Lactose (% w/w) | - | 4.85 |
| Fat (% w/w) | - | 0.05 |

Table 2 shows fermentation examples using acid cheese whey and the cement free alkali activated binder (examples 1-9), as well as examples of fermentation using acid cheese whey without said binder (examples 10-12).

**Table 2. Fermentation examples.**

| **Fermentation example** | **Inoculum, g** | **Acid cheese whey/binder, g** | **Acid cheese whey concentration, %** |
|---|---|---|---|
| | | | |
| 1 | 400 | 100/15 | 20 |
| 2 | 400 | 100/15 | 20 |
| 3 | 400 | 100/15 | 20 |
| 4 | 350 | 150/15 | 30 |
| 5 | 350 | 150/15 | 30 |
| 6 | 350 | 150/15 | 30 |
| 7 | 300 | 200/15 | 40 |
| 8 | 300 | 200/15 | 40 |
| 9 | 300 | 200/15 | 40 |
| 10 | 400 | 100/0 | 20 |
| 11 | 350 | 150/0 | 30 |
| 12 | 200 | 200/0 | 40 |
| 13 | 500 | 0/0 | 0 |

Table 3 shows amount of biogas obtained as a result of fermentation of acid cheese whey with the binder and without binder.

**Table 3. Amount of biogas obtained as a result of fermentation.**

| **Sample** | **Biogass, ml** |
|---|---|
| 20% acid cheese whey with binder | 4197 |
| 30% acid cheese whey with binder | 6222 |
| 40% acid cheese whey with binder | 8804 |
| 20% acid cheese whey w/o binder | 2064 |
| 30% acid cheese whey w/o binder | 5948 |
| 40% acid cheese whey w/o binder | 6934 |
| Reference | 1136 |

The experiments using cheese whey from a production plant have confirmed that the alkali activated, non-cement binders do serve as materials providing buffer capacity and provide no need to control the pH either in the beginning or during the process. The experiments showed possibility of using the cement free alkali activated binder for producing biogas not only from acid chese whey, but also from sweet cheese whey. The produced biogas can either be used for heating within the plant or transformed to electricity according to the wishes of the user.

### References:

1. Andini, S., Cioffi, R., Colangelo, F., Grieco, T., Montagnaro, F., Santoro, L., Coal fly ash as raw material for the manufacture of geopolymer-based products. Waste Management 2008, 28.. p. 416-423.
2. Bajare, D., Korjakins, A., Kazjonovs, J., Rozenstrauha, I. Pore structure of lightweight clay aggregate incorporate with non-metallic products coming from aluminium scrap recycling industry. Journal of the European Ceramic Society, Vol.32, No. 1, 2012. p. 141-148.
3. Brew, D.R.M., MacKenzie, K.J.D. Geopolymer synthesis using silica fume and sodium aluminate. J. Mater. Sci. 42. 2008. p. 3990-3993.
4. Deevasan, K.K., Ranganath, R.V. Geopolymer concrete using industrial by products. Proceedings of the Institution of Civil Engineers. Construction Materials, 2011, 164, CM1. p.43-50.
5. Ferchichi M, Crabbe E, Gil G, HintzW, Almadidy A. Influence of initial pH on hydrogen production from cheese whey. J Biotechnol 2005;120:402-9.
6. Fernando Pacheco-Torgal, João Castro-Gomes, Said Jalali (2008). Alkali-activated binders: A review: Part 1. Historical background, terminology, reaction mechanisms and hydration products. Construction and Building Materials. 22(7), 1305-1314
7. Fox P F, Guinee T P, Cogan T M and McSweeney P L H (2000) Fundamentals of Cheese Science. Gaithersburg, MD: Aspen
8. Gil A. Management of the Salt Cake from Secondary Aluminium Fusion Processes. Industrial Engineering Chemistry Research, 2005. 44(23), p.8852-8857.
9. Hardjito, D., Wallah, S.E., Sumajouw, D.M.J., Rangan, B.V. On the development of fly ash based geopolymer concrete. ACI Materials Journal 101(6). 2004. p. 467-472.
10. Hwang, J.Y., Huang, X., Xu, Z. Recovery of metals from aluminium dross and salt cake. Journal of Minerals & Materials Characterization & Engineering, 5(1),2006. p. 47-62.
11. Innovate with dairy, http://www.innovatewithdairy.com/Pages/FactsAboutWhey.aspx
12. Jasko J., Skripsts E., Dubrovskis V., Zabarovskis E., Kotelenecs V. "Biogas production from cheese whey in two phase anaerobic digestion." 10th International Scientific Conference Engineering for Rural Development, Jelgava (Latvia), 26-27 May 2011. 373-376.
13. Lucheva, B., Tsonev, T., Petkov, R. Non-waste Aluminium Dross Recycling. Journal of the University of Chemical Technology and Metallurgy. 2005. 40 (4), p. 335-338. Milk and milk products in the European Union, 2006, ISBN 92-79-02199.
14. Moulin, G. and Galzy, P. (). Whey, a potential substrate for biotechnology. Biotechnol Genet Eng Rev 1984, 1:347-373.
15. Mukhopadhyay, J., Ramana, Y.V., Singh, U. Extraction of Value Added Products from Aluminium Dross Materials to Achieve Zero Waste. Light Metals, 2005, p. 1209-1212.
16. Nasir, I., Mohd Ghazi T.I. and Omar R. Anaerobic digestion technology in livestock manure treatment for biogas production: A review. Engineering in Life Sciences Special Issue: Biogas, 2012, 12 (3), p 258-269.
17. Pennington, J., VanDevender K. and Jennings J.A. - Univ. of Arkansas Cooperative Extension Service, Division of Agriculture (FSA4017).
18. Phair, J.W., Van Deventer, J.S.J. Characterization of fly-ash-based geopolymeric binders activated with sodium aluminate. Ind. Eng. Chem. Res. 41. 2002. p.4242-4251.
19. Samuel, M. A new technique for recycling aluminium scrap. Journal of Materials Processing Technology 135(1). 2003. p. 117-124.
20. Shen H., Forssberg E. An overview of recovery of metals from slags. Waste Management 23. 2003. p. 933-949.
21. Shinzato, M.C., Hypolito, R. Solid Waste from Aluminium Recycling Process: Characterization and Reuse of its Economically Valuable Constituents. Waste Management, 2005. p. 37-46.
22. Speece, R. E. (1996). Anaerobic Biotechnology for Industrial Wastewaters. Vanderbilt University, Nashville, Tennesee: Archae Press.
23. Tzonev T.Z., Lucheva B. Recovering aluminium from aluminium dross in a DC Electric-ATC rotary furnace, Aluminium, JOM, November, 2007. p. 64-67.
24. Van Deventer, J.S.J., Provis, J.L., Duxson, P., Lukey G.C., J. Reaction mechanisms in the geopolymeric conversion of inorganic waste to useful products. Hazard.Mater. 139. 2007. p. 506-513.
25. Van Jaarsveld, J.G.S., Van Deventer, J.S.J., Lukey, G.C. The characterisation of source materials in fly ash-based geopolymers. Mater. Lett. 57. 2003. p.1272-1280.
26. Van Jaarsveld, J.G.S., Van Deventer, J.S.J., Lukey, G.C. The effect of composition and temperature on the properties of fly ash- and kaolinite-based geopolymers. Chem. Eng. J. 89, 2002. p. 63-73.
27. Venkata M. Anaerobic biohydrogen production from dairy wastewater treatment in sequencing batch reactor (AnSBR): Effect of organic loading rate, Enzyme and Microbial Technology. 41 (2007) 506-515
28. Xu, H., Van, Deventer, J.S.J. Effect of source materials on geopolymerization, Ind.Eng. Chem. Res. 42. 2003. p.1698-1706.
29. Yoshimura, H.N., Abreu, A.P., Molisani, A.L., de Camargo, A.C., Portela, J.C.S., Narita, N.E. Evaluation of aluminium dross waste as raw material from refractory. Ceramic International, 34(3). 2008, p. 581-591.

## Claims

1. Use of a cement free alkali activated binder as pH stabilizer and/or material for cell immobilization for producing biogas from cheese whey, wherein said binder comprises alumo-silicate source materials, lead-silicate glass, calcinated metakaolin clay, and alcaline inorganic materials comprising sodium materials.

2. Use of the binder according to claim 1, wherein alumo-silicate source materials comprise aluminium recycling waste; and the lead-silicate glass is being recycled lead-silicate glass.

3. A cement free alkali activated binder intended for use for producing biogas from cheese whey, the binder comprising alumo-silicate source materials, lead-silicate glass, calcinated metakaolin clay, and alcaline inorganic materials comprising sodium materials, wherein alumo-silicate source materials comprise:
metallic aluminium (Al) in the amount of 3-15 weight %,
aluminium oxide (Al₂O₃) or aluminium hydroxide (Al(OH)₃) - 20-50 weight %,
aluminium nitride (AlN) - 3-15 weight %,
silica (SiO₂) - 3-30 weight %,
ferrous III oxide (Fe₂O₃) - 2-20 weight %,
ferrous II oxide (FeO) - 2-20 weight %,
magnum oxide (MgO) - 1-20 weight %,
calcium oxide (CaO) - 1-20 weight %.

4. The binder according to claim 3, wherein said alcaline inorganic materials comprise alkali flakes (NaOH), liquid glass (Na₂SiO₃+nH₂O) and liquid type sodium hydroxyl flakes.

5. The binder according to claim 4, wherein the amount of said alkali flakes is from 7 to 12 weight % from the weight of water glass.

6. A method for producing biogas from cheese whey using cement free alkali activated binder, comprising the following steps:
(i) providing fermentation medium containing between 50 weight % and 80 weight % of inoculum, as well as between 20 weight % and 50 weight % of cheese whey,
(ii) adding 10-20 weight % of a cement free alkali activated binder,
(iii) performing fermentation,
(iv) collecting the produced biogas.

7. The method according to claim 6, wherein the cement free alkali activated binder comprises alumo-silicate source materials, lead-silicate glass, calcinated metakaolin clay, and alcaline inorganic materials comprising sodium materials.

8. The method according to claim 6 or 7, wherein cheese whey FOS/TAC in the fermentation medium is between 3000 mg/L and 13000 mg/L.

9. Biogass obtained by the method according to any claims 6-8.
